# EUROPEAN PATENT SPECIFICATION

(11) **EP 1 292 320 B1**
(45) Date of publication and mention of the grant of the patent: **16.11.2005**
(21) Application number: 01934247.6
(22) Date of filing: 29.05.2001
(51) Int. Cl.: A61P 37/04, A61P 37/06, A61K 35/78

(54) **USE OF EXTRACTS FROM SPERMATOPHYTE PLANTS WITH IMMUNOMODULATING ACTIVITY**
VERWENDUNG VON SPERMATOPHYTE PFLANZENEXTRAKTE MIT IMMUNOMODUELIERENDER WIRKUNG
UTILISATION D'EXTRAITS DE SPERMATOPHYTES COMME MODULATEURS IMMUNITAIRES

(30) Priority: 30.05.2000 IT RM000294
(43) Date of publication of application: 19.03.2003
(73) Proprietor: CONSIGLIO NAZIONALE DELLE RICERCHE, I-00100 Roma (IT); Istituto Agrario di S. Michele All'Adige, 38010 S. Michele All'Adige (IT)
(72) Inventor: RAVAGNAN, Giampietro, I-00164 Roma (IT); FALCHETTI, Roberto, I-00124 Roma (IT); LANZILLI, Giulia, I-00141 Roma (IT); FUGGETTA, Maria, Pia, I-00179 Roma (IT); TRICARICO, Maria, I-00143 Roma (IT); MATTIVI, Fulvio, I-38042 Baselga di Pinè (IT)
(74) Representative: Gervasi, Gemma
(86) International application number: PCT/IB2001/000983
(87) International publication number: WO 2001/091764

(56) References cited:
- WO-A-01/03713
- WO-A-01/30336
- WO-A-99/58119
- MATTIVI FULVIO ET AL: "Isolation, characterization, and evolution in red wine vinification of resveratrol monomers." JOURNAL OF AGRICULTURAL AND FOOD CHEMISTRY, vol. 43, no. 7, 1995, pages 1820-1823, XP002197355 ISSN: 0021-8561
- KORHAMMER SIEGFRIED ET AL: "An oligostilbene from Vitis roots." PHYTOCHEMISTRY (OXFORD), vol. 38, no. 6, 1995, pages 1501-1504, XP000946048 ISSN: 0031-9422
- TRELA B C ET AL: "RESVERATROL: ISOMERIC MOLAR ABSORPTIVITIES AND STABILITY" JOURNAL OF AGRICULTURAL AND FOOD CHEMISTRY, AMERICAN CHEMICAL SOCIETY. WASHINGTON, US, vol. 44, no. 5, 1 May 1996 (1996-05-01), pages 1253-1257, XP000583846 ISSN: 0021-8561
- OHYAMA MASAYOSHI ET AL: "Antitumor agents 200. Cytotoxicity of naturally occurring resveratrol oligomers and their acetate derivatives." BIOORGANIC & MEDICINAL CHEMISTRY LETTERS, vol. 9, no. 20, 18 October 1999 (1999-10-18), pages 3057-3060, XP002196352 ISSN: 0960-894X
- CHEONG, HO ET AL: "Anti-allergic action of resveratrol and related hydroxystilbenes" PLANTA MED. (1999), 65(3), 266-268, XP001032461
- GIOVANNINI, L. ET AL: "Pretreatment with resveratrol, a natural compound from grapes and wine, decreases ischemia/reperfusion-induced polymorphonuclear cell infiltration in rat kidney" INTERNATIONAL JOURNAL OF IMMUNOTHERAPY (2000), 16(1/2), 19-26 , XP001069872
- KIMURA Y ET AL: "Effects of naturally occurring stilbene glucosides from medicinal plants and wine, on tumour growth and lung metastasis in Lewis lung carcinoma-bearing mice." JOURNAL OF PHARMACY AND PHARMACOLOGY, (2000 OCT) 52 (10) 1287-95. , XP001069866
- GAO X ET AL: "Immunomodulatory activity of resveratrol: suppression of lymphocyte proliferation, development of cell-mediated cytotoxicity, and cytokine production." BIOCHEMICAL PHARMACOLOGY, (2001 NOV 1) 62 (9) 1299-308., XP002197358
- FALCHETTI R ET AL: "Effects of resveratrol on human immune cell function." LIFE SCIENCES, (2001 NOV 21) 70 (1) 81-96., XP002197359

## Description

### Field of the invention

The present invention relates to a method for the extraction of pharmaceutically active products from spermatophyte plants, to the products thus obtained and to their use in the medical field, in particular as substances with immunomodulating activity.

In particular, the invention relates to the use in the pharmaceutical field with immunomodulating activity of cis or trans resveratrol, (indicated below as C-Res and T-Res respectively) and of hydroxylated stilbenes, oligostilbenes and stilbenoids both in their free and glucosidated forms.

### State of the art

The immune system protects the organism from the aggressions of various pathogenic agents, such as virus, bacteria, mycoplasma, fungi and protozoa, from foreign substances and from the development of carcinogenic cells thanks to humoral and cell factors. However, immune reactions do not always play a positive role, since they can also raise reactions of hypersensitivity and autoimmunity.

In recent years the results of a series of researches have shown the possibility to modulate and control immune responses. At present the stimulation and suppression of the immune system are used in the treatment or prevention of various pathologies.

Immunomodulators are compounds modifying immune functions which can act positively or negatively on the activity of the immune system. Said class of drugs comprises chemical compounds, such as for instance organic compounds; substances of biological origin or molecules of natural origin.

In the medical practice the use of immunomodulators comprises both the stimulation or reconstitution of immune response (correction of immunodeficiency) and the suppression of normal or excessive immune response. The stimulation of the immune system is important for instance to protect a patient from tumors or to increase the immune response in patients having a defective immune system, such as surgery and burnt patients, patients undergoing radiotherapy or chemotherapy and patients suffering from AIDS. In general, these immunodefective patients can develop infections from virus such as cytomegalovirus, herpetic virus, syncytial respiratory virus and hepatitis virus.

Immunomodulators can be used to stimulate the immune response to said infections or as prophylactic agents in their prevention. Moreover, said substances can also be used in the treatment of autoimmune illnesses.

One of the key mechanisms in the immune response is the production by T lymphocytes of cytokines, a group of polypeptides having a hormone-similar effect and affecting the function of various kinds of cells. Recent researches have shown that the production of cytokines by CD4 positive T cells (CD4+) and CD8+ T cells often falls into one of the two phenotypes TH1 and TH2. TH1 cells produce interleukine 2 (IL2), interferon-g (IFN-g) and tumor necrosis factor (TFN) and are mainly responsible for cell-mediated immunity, such as for instance retarded hypersensitivity. TH2 cells produce other types of interieukines such as IL4, IL5, IL6, IL9, IL10 and IL13 and are mainly responsible for the regulation of tumoral immunity.

Strongly polarized TH1 and TH2 responses play an important role, beyond the protection of the organism from infective agents or tumor cells, in the induction of various pathologies, such as for instance organ-specific autoimmunity and some types of chronic inflammation as far as TH1 responses are concerned; allergic asthma and atopical dermatitis as far as TH2 responses are concerned. Anyway, the two types of response are strictly related one to the other, since the stimulation of TH1 response induces in most cases an inhibition of the production of TH2 cytokines and vice versa.

It is therefore evident that the modulation of the production of cytokines by T cells can play a fundamental role in the control of various pathologies, and substances which can modulate said production can have an important immunopharmacological and therapeutic effect.

Hydroxylated stilbenes, both monomer and oligomer, represent a class of chemical compounds which are present in a limited number of spermatophyte plants and in particular in vine, where they are essential components of the root of leaf stems and mainly of fruits (Vrhovsek U, Mattivi F, 1998, *Proceedings of the 29*^{*th*} *J. Plecnik, Cardiovascular Diseases*, 449-463; Mattivi F et al., 1995, *J Agric Food Chem*, 42, 1820-1823). As their role in plant physiology seems to be mainly the inhibition of the progression of infections caused by fungi, this group of substances has been included among phytoalexins, a class of antibiotics of vegetal origin (Hain R et al, 1990, *Plant Mol Biol*, 15, 325-335).

The results of a series of researches made by several groups of researchers indicate that one of the compounds belonging to the class of stilbenes, trans-resveratrol, can perform pharmacological activities in humans. Indeed, these researches have shown that trans-resveratrol has anti-oxidant properties (Fauconneau B et al, 1997, *Life Sci*, 61, 2103-2110), inhibits platelet aggregation (Bertelli A et al, 1996, *Drug Exp Clin Res,* 22, 61-63) and cyclo-oxygenase activity (Jang M et al, 1997, *Science*, 275, 218-220). Moreover, it has been proved that said compound can inhibit *in vitro* the growth of cells belonging to line MCF-7 derived from mammary adenocarcinoma (Mgbonyebi O P et al, 1998, *Int J Oncology*, 12, 865-869), and in cells belonging to line HL60 (human promyelocytic acute leukemia) it can induce the stop of the cellular cycle in the transition from stage S to stage G2 (Della Ragione F et al, 1998, *Biochem Biophys Res Com*, 250, 53-58), and in high doses it can induce apoptosis and regulate the expression of CD95L (Clement MV et al, 1998, *Blood*, 92, 996-1002). Finally, *in vivo* trans-resveratrol has proved to be able to inhibit tumor genesis in a murine model of skin cancer induced by carcinogenic substances (Jang M et al, 1997, Science, 275, 218-220).

During our researches we have surprisingly found that, in addition to the aforesaid activities, trans- and cis-resveratrol can modulate both natural killer response (NK) and antigen-specific cytotoxic lymphocytic response (CTL). Furthermore, we have found that the aforesaid substances can modulate the production of cytokines by CD4+ and CD8+ T cells.

### Summary of the invention

The present invention is therefore based on the finding that hydroxylated stilbenes, in particular cis- and trans-resveratrol, can modulate the effect-producing response of human T lymphocytes and can therefore be used as drugs for modulating the response of the immune system in various pathologic situations, such as for instance i) primary immunodeficiency in children and in adults; ii) secondary immunodeficiencies deriving for instance from undernourishment, lymphoproliferative illnesses, infections (induced by virus, bacteria, fungi, animal parasites), surgery interventions, radiotherapy or chemotherapy treatments, drug administration, burns and nephrotic syndrome; iii) autoimmune illnesses such as for instance rheumatoid arthritis, uveitis, psoriasis; iiii) transplant reject.

Another object are the products of the extraction process, which are used in the pharmaceutical field, said products consisting in complex mixtures comprising compounds having in their molecule one or more stilbene groups, variously hydroxylated and/or glucosidated, and compounds resulting thereof by natural enzymatic biosynthetical processes (oligostilbenes are defined as those oligomers which have in their molecule at least a recognizable stilben bond, and stilbenoids those oligomers which have involved all stilbene double bonds in the condensation process). Preferred compounds are the following:
An object of the invention is the use of the compounds T-Res, C-Res, glucosidated C-Res, ε-viniferina, H gnetine, r-2-viniferine, r-viniferine, hopeaphenol, Ampelopepsin A and glucosidated T-Res, in particular as drugs with immunomodulating activity, more in particular as 1) immunomodulating drugs to be used in various pathologies, such as for instance i) primary immunodeficiency in children and in adults; ii) secondary immunodeficiencies deriving for instance from undernourishment, lymphoproliferative illnesses, infections (induced by virus, bacteria, fungi, animal parasites), surgery interventions, radiotherapy or chemotherapy treatments, drug administration, burns and nephrotic syndrome;
2) immunosuppressing drugs to be used in pathologies such as for instance i) autoimmune illnesses (for instance rheumatoid arthritis, uveitis, psoriasis); ii) transplant reject.

An additional object of the present invention is the use of the compounds deriving from the extraction in different concentrations according to the immunostimulating and immunodepressive activity.

### Brief description of the figures

Fig. 1 A shows the effect of resveratrol on the production of cytokines by CD8+/IFN+ T cells activated with anti-CD3/CD28.
Fig. 1B shows the effect of resveratrol on the production of cytokines by CD8+/IL2+ T cells activated with anti-CD3/CD28.
Fig. 1C shows the effect of resveratrol on the production of cytokines by CD8+/IL4+ T cells activated with anti-CD3/CD28.
Fig. 2A shows the effect of resveratrol on the production of cytokines by CD4+/IFN+ T cells activated with anti-CD3/CD28.
Fig. 2B shows the effect of resveratrol on the production of cytokines by CD4+/IL2+ T cells activated with anti-CD3/CD28.
Fig. 2C shows the effect of resveratrol on the production of cytokines by CD4+/IL4+ T cells activated with anti-CD3/CD28.
Fig. 3A shows the effect of resveratrol on the replication of lymphocytes activated with anti-CD3/CD28.
Fig. 3B shows the effect of resveratrol on DNA synthesis of lymphocytes activated with anti-CD3/CD28.
Fig. 4A shows the effect of resveratrol on the induction of apoptosis in lymphocytes activated with anti-CD3/CD28.
Fig. 4B shows the effect of resveratrol on the mortality of lymphocytes activated with anti-CD3/CD28.
Fig. 5A shows the effect of resveratrol on the Natural Killer (NK) activity.
Fig. 5B shows the effect of resveratrol on the expression of surface antigens CD16 and CD95 in human lymphocytes.
Fig. 5C shows the effect of resveratrol on the expression of surface antigens CD4 and CD95 in human lymphocytes.
Fig. 6A shows the effect of resveratrol on the cytotoxic activity of T lymphocytes.
Fig. 6B shows the effect of resveratrol on the blastization of T lymphocytes.

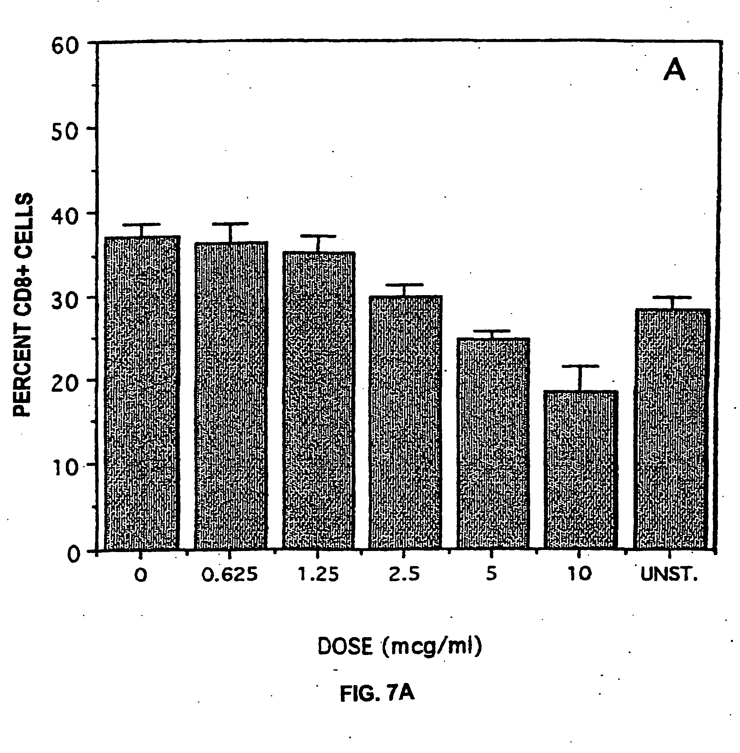

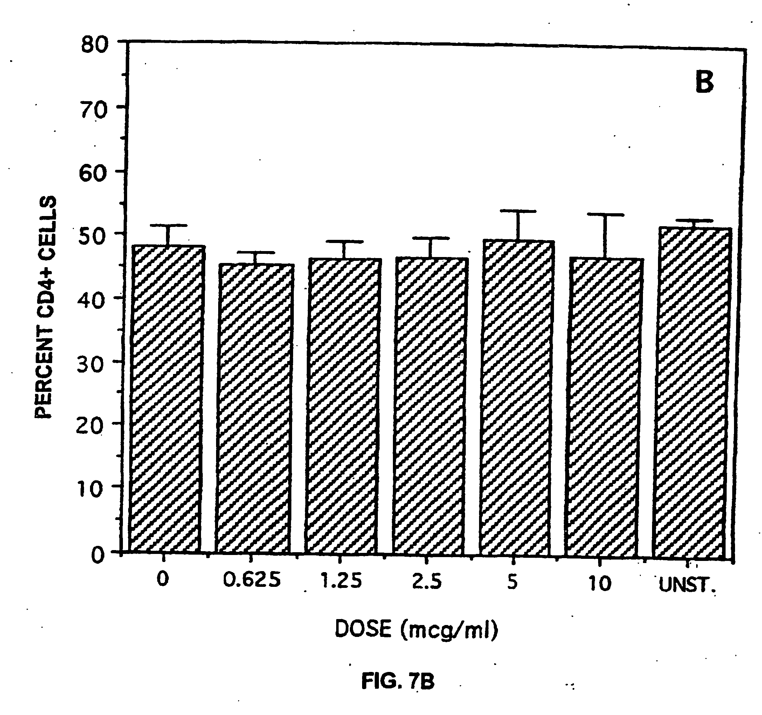

Fig. 7A shows the effect of resveratrol on the percentage of CD8+ cells.
Fig. 7B shows the effect of resveratrol on the percentage of CD4+ cells.

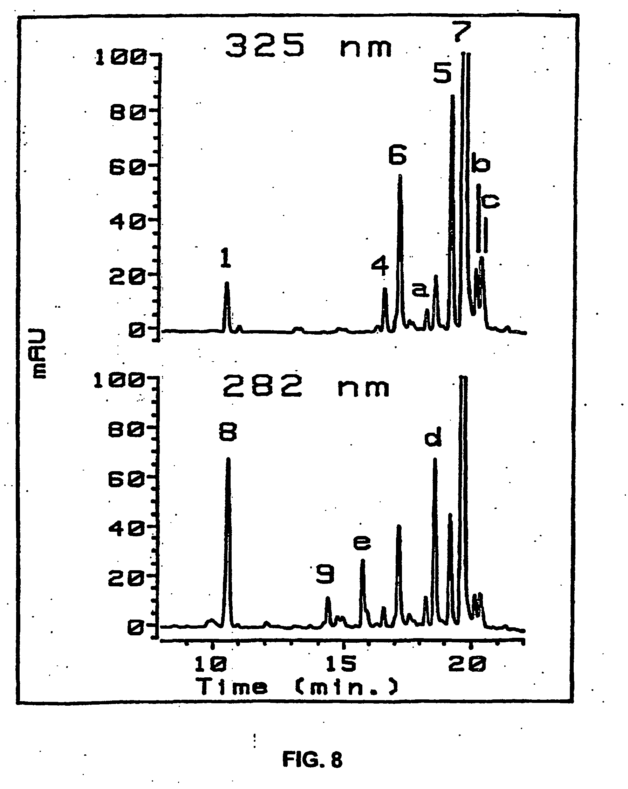

Fig. 8 shows the reversed-phase HPLC chromatographic plot of the complex mixture of stilbenes, oligostilbenes and stilbenoids extracted from Vitis roots: trans-stilbenes and trans-oligostilbenes are monitored at 320 nm, cis-stilbenes and stilbenoids at 282 nm.

### Detailed description of the invention:

The compounds used in the present invention can be roughly divided into two families: the first is the one of stilbenes and oligostilbenes, the second is the one of stilbenoids. The first one comprises molecules characterized by the presence of one or more stilbene groups [(C₆H₅)-CH=CH-(C₆H₅)], variously hydroxylated and/or glucosidated. As a mere non-limiting example, the following compounds belong to said first family: 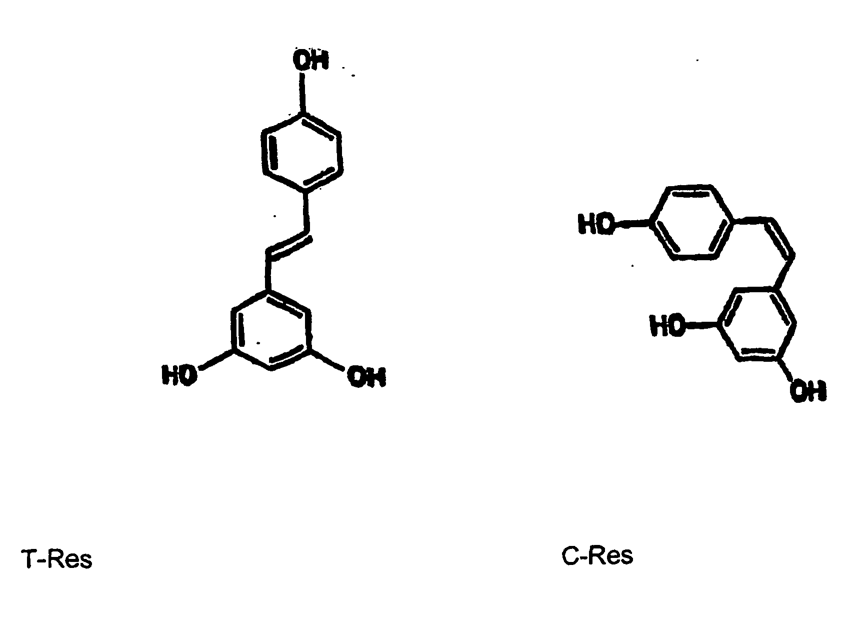 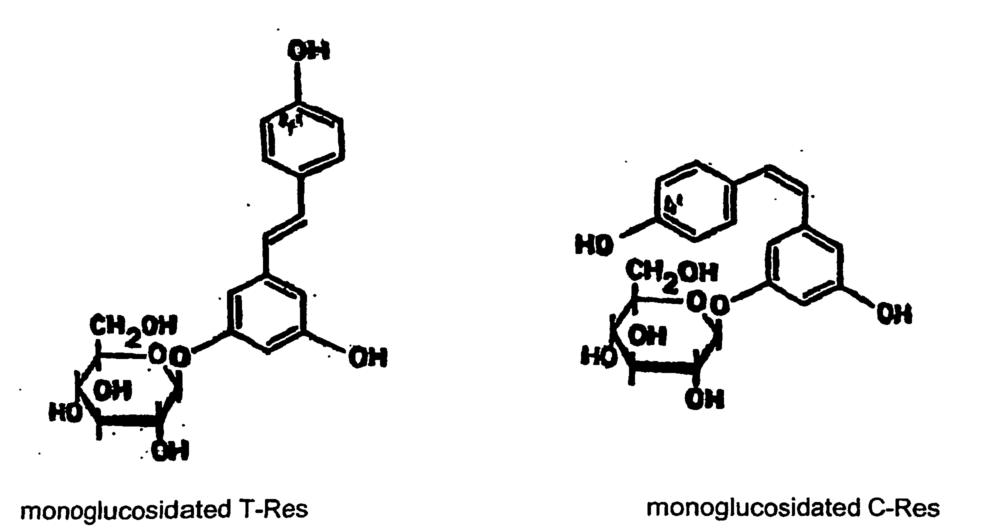 (alternatively to position 3, the glucosidic bond can also be present in position 4' and result in different T-Res and C-Res, differently monoglucosidated, or T-Res and C-Res having more than one glucosidic group).

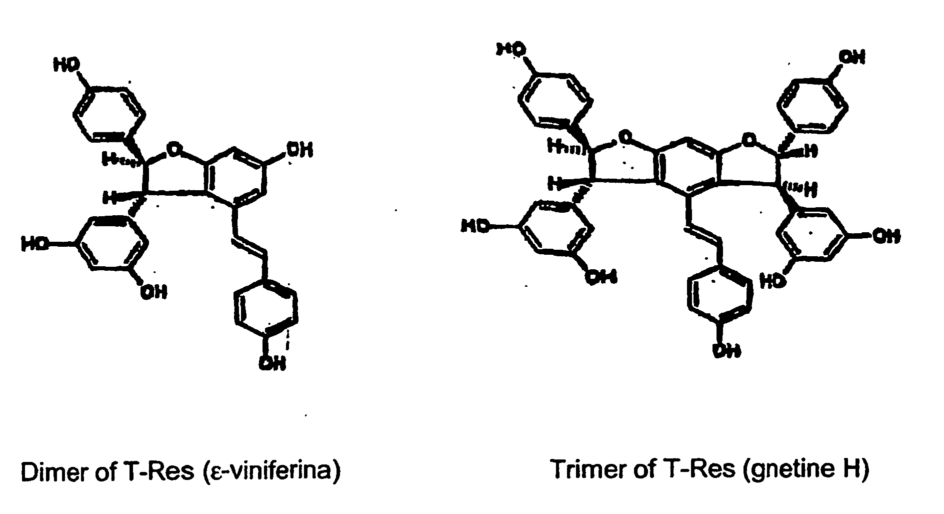

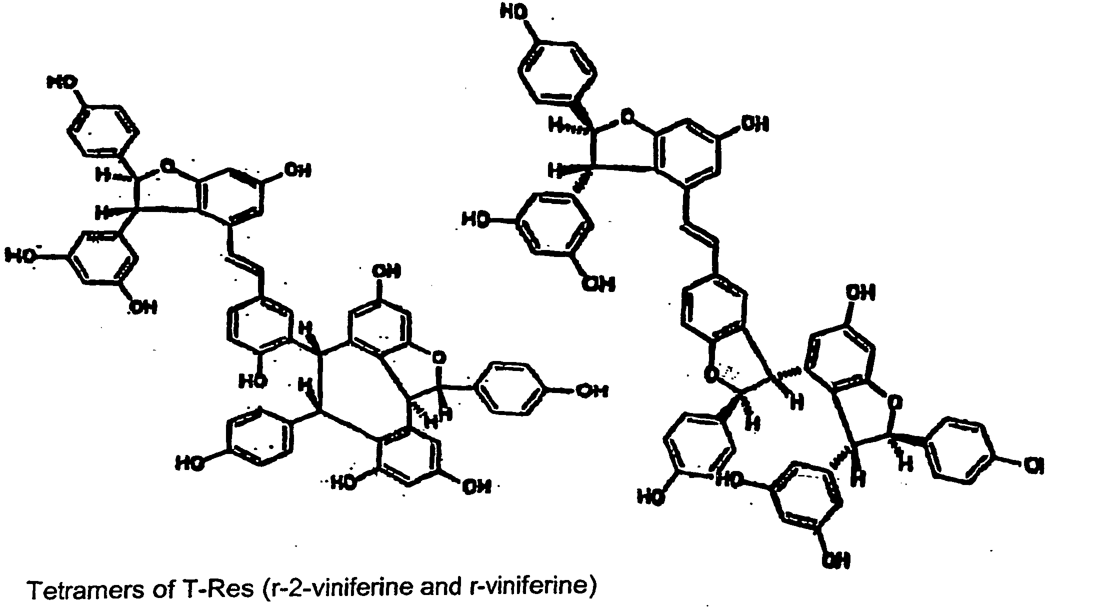

The second family, the one of stilbenoids, comprises compounds which, like the previous ones, can be classified as stilbene oligomers, but in which the original stilbene structure is no more recognizable, since all stilbene double bonds have been modified by natural enzymatic biosynthetical processes within the vegetal products they are extracted from.

As a mere non-limiting example the following compounds can be mentioned:

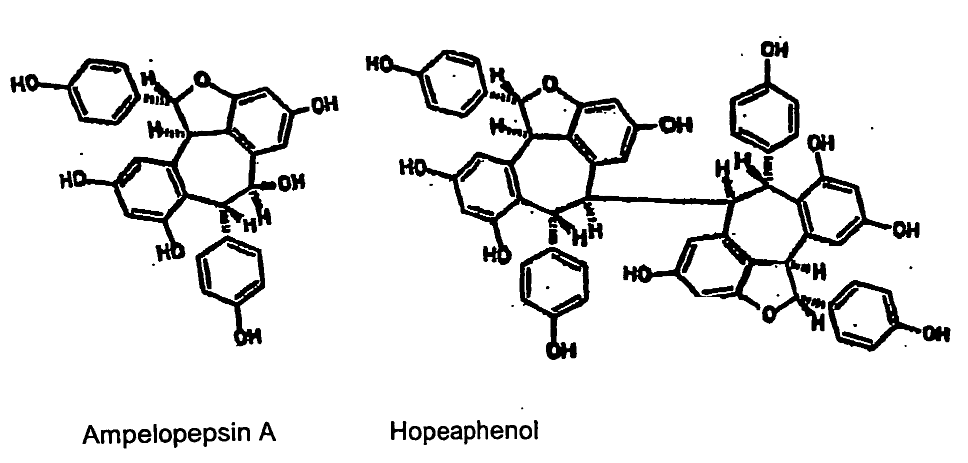

Ampelopepsin A is considered as an oxidative dimer of T-Res. Hopeaphenol is the corresponding tetramer, i.e. a dimer of dimers which are bonded through two C8 positions.

The compounds belonging to the two classes can be obtained by means of extraction and purification treatments from the natural products containing them, for instance fruits (ex. grapes), aerial parts (trunk, shoots, leaves) or subterranean parts of spermatophyte plants, in particular those belonging to the family of Vitaceae or Polygonaceae. Subterranean parts of such plants are particularly preferred.

Extraction and purification process follow the methods indicated below. It should be kept in mind that the mixtures obtained with the process according to the present invention are complex mixtures within which only the main components have been identified (stilbenes, oligostilbenes and stilbenoids indicated above): however, these products have to be regarded only as representative of other similar components, which are present in smaller quantities and which have not been isolated and characterized though they have the same nature and features of applications; these products are also part of the present invention, though not isolated and characterized, since they are present in natural extracts.

The present invention refers to the immunomodulating activity carried out by these compounds, both alone and in mixture.

The extraction process according to the present invention is applied to spermatophyte plants. The starting material can be fruits, aerial parts or subterranean parts of the plants. The starting material can be fresh or frosted or lyophilized and pulverized. The most significant matrixes are considered to be the roots of Polygonum cuspidatum and Polygonum multiflorum and the bark of lignified root of the genus Vitis.

It is preferable to work with materials which are as dry as possible, so that it can be advantageous to subject said material to be extracted to a pre-treatment including the substantial cold elimination of water, for instance by lyophilization. The extraction is carried out in a neutral environment with aliphatic alcohol, preferably methanol or ethanol and their mixtures, preferably using solvent amounts between 10 to 20 times by volume the weight of the matrix to be extracted. The material to be extracted and the solvent are mixed, the whole is agitated and extracted in oxygen-free atmosphere, for instance saturated with nitrogen, and light-shielded, at room temperature, with a duration of the extraction varying according to the matrix, generally around 2-12 hours for matrixes as such, and around 5-120 minutes for matrixes which have been previously lyophilized and pulverized by previously grinding the lyophilized product. The final extract is centrifuged and the supernatant liquor is recovered, the latter is concentrated under vacuum at low temperatures (lower than 45°C) and taken up with ethyl acetate or another similar solvent such as for instance methyl acetate and/or tetrahydrofuran (raw extract in solvent) or with water (aqueous raw extract) (in particular for Polygonum roots, so as to obtain a higher purity degree).

The raw extract can be treated following one of two methods, depending whether a mixture substantially containing all the products according to the present invention or only trans-resveratrol and glucosidated trans-resveratrols is to be obtained.

The first kind of treatment (Treatment A) is preferred in case the extraction is carried out on plants of the genus Vitis, whereas the second kind of treatment (Treatment B) is preferred for the simpler matrix related to plants of the genus Polygonaceae.

Treatment (A) The raw extract in a solvent such as ethyl acetate is washed with water saturated with an inorganic salt (for instance NaCl), the fraction in ethyl acetate is loaded onto a column prepared with a resin of an aromatic polymer (stirene-divinylbenzene copolymers, preferably with grain size between 0.1 and 0.25 mm, are particularly suitable to this purpose).

After the loading the whole is eluted with water, preferably in a volume which is about twice that of ethyl acetate, then with pentane-methylene chloride 2:1 (in a volume which is almost as much as that of ethyl acetate). Stilbenes are eluted with ethyl acetate or with suitable mixtures of organic solvents with intermediate polarity, whose eluting strength corresponds to XAD-2, an abbreviation which is known to the person skilled in the art. XAD-2 is a particular kind of stirene-divinylbenzene resin with respect to which the factors concerning relative eluting strength are tabulated in the scientific literature (see e.g. Robinson J.L. et al., *J.*

*Chromatogr.*, 1980, 185, 145), such as for instance tetrahydrofuran and methylene chloride. The volume of ethyl acetate is chosen so as to ensure the quantitative recovery of the whole class (such as in Fig. 14) and varies according to the shape and free volume of the column. The product of this selective elution consists of a purified fraction containing the whole class of viniferine, containing both oligostilbenes and stilbenoids.

The basic constituents ensuring a pharmacological interest to this fraction are the oligomers of resveratrol, with particular attention to dimers, trimers and tetramers, both those still containing a stilbene double bond in trans or cis form (oligostilbenes) and those which have lost their stilbene structure during natural polymerization (stilbenoids). The same fraction also contains glucosidated trans-resveratrol.

If the following pure compounds are to be obtained: epsilon-viniferine, alfa-viniferine, H-gnetine (which can also be isolated from wood of Welwitschia mirabilis),r-viniferine, Ampelopepsin A (which can also be isolated from roots of Ampelopsis brevipedunculata) and hopeaphenol, a high-performance liquid chromatography on reversed-phase columns has to be carried out. Phases based on silica functionalized with C18 o C8 (terms known to the person skilled in the art), or stirene polymers can be used, the latter only with low pressure. A practical example (ideal to separate epsilon-viniferine, H-gnetine and r-viniferine) is the use of a packed column with fixed phase RP-18, for instance LiChrospher 100 or similar, 10 micron of particle size, eluting with a linear gradient of water and acetonitrile, the latter 30 to 50%. The setup of the separation conditions for this kind of mixtures is known to the person skilled in the art.

A quantitative analysis of the mixture of stilbenes, oligostilbenes and stilbenoids obtained with the extraction according to the invention can be generally obtained by reversed-phase high-performance liquid chromatography (or with other separation techniques in liquid phase such as electrophoresis, thin layer chromatography (TLC) with UV detection, MS (Mass Spectrometry) or fluorescence detector. The first two techniques are preferred since they allow a reliable identification. An example of optimized conditions can be found in example 8.

### Treatment B

Treatment B consists of the following alternatives:
- if both lipophilic and hydrophilic compounds have to be eliminated from the final extract, the aqueous raw extract is washed with methylene chloride or other solvent with similar polarity such as for instance chloroform, in order to remove lipophilic products though not stilbenes, and is then re-extracted in ethyl acetate (for instance five times with extract/extractant volume of 1/1, to be reduced in case of higher volumes of extractant or of modification of the ionic strength of the extract), in order to recover stilbenes, whereas hydrophilic compounds remain in the water portion which is discarded.
- if only glucosidated derivatives have to be obtained from the final extract, these can be selectively cold-precipitated from the raw extract in a solvent such as ethyl acetate with a non polar solvent (hexane would be ideal, in a 3/1 ratio with respect to ethyl acetate), and recovered by filtration. Alternatively, the whole fraction can be used in order to recover trans-resveratrol as well.
- if a quite accurate separation of the mixture has to be obtained, it is possible to start from the raw extract in a solvent such as ethyl acetate, which should be perfectly anhydrous. Said raw extract, preferably reduced to its minimum volume, is loaded into the head of a preparative silica column for chromatography (for instance Kieselgel 0.05-0.20 mm), the latter being packed in a non polar solvent (for instance hexane). A suitable volume of chloroform is left above the column.

The treatment provides for two subsequent washing and elution sequences with mixtures having increasing eluting strengths, chloroform-methanol, I (20:1); II (10:1); III (5:1); IV (2.5:1) respectively. More generally, with suitable binary or ternary mixtures of solvents with moderate polarity (for instance chlorinated solvents, diethyl ester, tetrahydrofuran) and strong solvents (for instance aliphatic alcohols, acetonitrile) it is possible to obtain similar separations using mixtures having a position in the elution series which is wholly similar to the ones indicated above, according to what is known to the person skilled in the art. This separation enables an integral recovery of stilbene active agents, separated from all major interfering substances, thus obtaining two preparations with very high purity: fraction IV contains two different monoglucosidated derivatives of trans-resveratrol, gathered into the same fraction, and fraction II contains free tansresveratrol. The conditions here described refer in particular to the roots extract of Polygonum cuspidatum, and can be adapted with a similar procedure to Polygonum multiflorum, which contains - as is known - also other kinds of glucosidated stilbenes (2,3,5,4'-tetrahydroystilbene-2-glucoside, Yong et al., 2,2-*diphenyl-1-picriylhydrazyl radical-scavenging active components from polygonum multiflorum Thunb., J. Agr. Food Chem*., 1999, 47, 226-2228). If it is necessary to go as far as the molecular level, purification techniques by preparative chromatography are used, in the conditions described in Mattivi et al., *Isolation, characterization and evolution in red wine vinification of resveratrol monomers, J. Agr. Food Chem.*, 1995, 43, 7, 1820-1823.

A quantitative analysis of resveratrols can be carried out according to example 8.

The techniques for quantitative analysis described are particularly indicated for instance to evaluate the presence of the desired active agent in culture mediums, and therefore to support in vitro experimental models.

### Trans-cis isomerization

An indication of isomerization techniques according to the invention can be found in example 7.

### Therapeutic activity

The most important function of the immune system is to protect the organism from illnesses. In order to achieve said task, during its evolution the immune system has developed various effectors-producing mechanisms, both humoral and cell-mediated. Each of these effectors-producing mechanisms has unique features as far as its ability to affect progression speed or to promote the elimination of pathogenic microorganisms or tumor cells is concerned. Said variety of mechanisms is absolutely necessary since no effectors-producing response alone can face all kinds of pathogenic situations. Moreover, a suitable effectors-producing response should act selectively on the target organ and should be able to inhibit the development of other non-specific types of immune response in order to reduce the risk of pathologic consequences.

In the framework of cell-mediated responses two cell compartments can be distinguished: the first one concerns macrophages and natural killer cells (NK) and provides for a natural response, independent from the antigenic stimulus. The second one, related to acquired responses, concerns cytotoxic T lymphocytes (CTL) and provides for a response which is strictly related to the recognition of the antigen in association with the major histocompatibility complex (MHC). T lymphocytes, thanks to their ability to produce different types of cytokines as a response to various stimuli activating them, play a central role in the development of a correct effector-producing response by the immune system.

Therefore, pharmacological interventions modulating the immune system both by controlling directly the effector-producing response and by regulating synthesis and secretion of cytokines by T cells can have a direct quantitative and qualitative influence on the nature of the immune response.

The present invention describes a method for modulating, by means of the compounds according to the invention, in particular by means of resveratrol, the effector-producing activity of cells of the immune system and in particular the natural killer (NK) activity, the antigen-specific cytotoxic activity of T lymphocytes, the production of cytokines of TH1 and TH2 type by activated CD8+ and CD4+ T cells.

The effects of the immunomodulators described in the present invention on the functional parameters of the immune system mentioned above vary according to the dose of the administered substance (immunostimulating effect at low doses and immunosuppressive effect at higher doses). Therefore, the compounds according to the invention can be used both as immunostimulating drugs to strengthen the immune response in case of primary or secondary immunodeficiences, such as those present in patients suffering from tumors, AIDS, viral and bacterial infections or in old patients, and as immunosuppressive drugs in the treatment of autoimmune illnesses or in general to inhibit unwanted immune responses as in the case of asthma or transplant reject.

The immunomodulators described in the present invention can be used in combination with other pharmaceutically active therapeutic agents, such as for instance anti-tumor compounds (antimetabolites, intercalating agents, mitosis inhibitors or other cytotoxic inhibitors (for instance, platinum coordination complexes, vinca alkaloids, urea substitutes, L-asparaginase, corticoadrenal inhibitors). In the therapy extracts can be used as such, i.e. the individual compounds both in natural form (as isolated from the matrixes of vegetal origin) and as obtained from chemical synthesis. The active agent can be administered in form of pharmaceutically acceptable salt, ester, amide, prodrug or similar or their combinations. Salts, esters, amides, prodrugs or similar of the active agents can be prepared by following the standard procedures of organic synthetic chemistry.

On the basis of the administration way which is going to be used, the pharmaceutical formulation can be in form of tablets, suppositories, pills, capsules, powders, liquids, suspensions, creams, ointments, lotions or similar. The compositions will therefore include an effective amount of the agent in combination with a pharmaceutically acceptable excipient and can also include other pharmacological agents, adjuvants, diluents, buffers, etc. The compounds can then be administered by oral, parenteral (subcutaneous, intravenous, intramuscular injection), transdermic, rectal, nose, mouth, topical administration or by means of a controlled-release implant.

The amount of the active compound to be administered will depend on the particular pathology (or pathologies) affecting the patient to be treated, on the weight and age of the patient, on the chosen administration way and on the physician's opinion. In the method according to the invention, immunomodulating activity, the treatment scheme will provide for the administration of the drug at doses between 0.0001 and 20 mg/kg/die.

The clinical applications of the compounds will concern the prevention and therapy of pathologies whose solution requires the stimulation of the immune response, or its inhibition. Therefore, the compounds can be used: 1) as immunomodulating drugs in pathologies such as for instance i) primary immunodeficiency in children and in adults; ii) secondary immunodeficiencies deriving for instance from undernourishment, lymphoproliferative illnesses, infections (such as those induced by virus, bacteria, fungi, animal parasites), surgery interventions, radiotherapy or chemotherapy treatments, drug administration, burns and nephrotic syndrome; 2) as immunosuppressing drugs to be used in pathologies such as for instance i) autoimmune illnesses (for instance rheumatoid arthritis, uveitis, psoriasis); ii) transplant reject.

The following examples 1-16 are not part of the present invention.

### Example 1

### Extraction of resveratrols (trans-resveratrol, cis-resveratrol and their glucosides) from vegetal matrixes (fruits, vine grapes)

The matrix is frozen and then homogenized in presence of sodium metabisulfite and ascorbic acid in an amount of 1% by weight each, the homogenized product undergoes liquid-liquid extraction three times with ethyl acetate (150% by volume with respect to the matrix weight) in absence of light. The extracts are washed once with a 3% water solution of sodium bicarbonate and twice with distilled water, with a volume of 10% of the extract volume for each washing. The washed extract is anhydrified with anhydrous sodium sulfate or by freezing and concentrated to dryness under reduced pressure and at low temperature. The dry extract is taken up with anhydrous ethyl acetate.

### Example 2

### Extraction of resveratrols (trans-resveratrol, cis-resveratrol and their glucosides) from root of Polygonum cuspidatum

The root of the repotted plant is washed, dried, cut into big pieces, lyophilized and ground. The extraction takes place with methanol (or ethanol) under stirring in absence of light and oxygen. Then the extract is centrifuged and the supernatant liquor is recovered, the latter being concentrated under low pressure and temperature and taken up with ethyl acetate.

### Example 3

### Extraction of viniferine (oligomer stilbenes and stilbenoids) from vegetal matrixes

The extraction of viniferine from aerial parts (trunk, shoots, leaves) or from subterranean parts of plants of the family of Vitaceae or Polygonaceae can be carried out both on fresh or frozen matrix, or on the lyophilized and pulverized part. If the extraction is carried out on the roots, the latter are washed, dried, cut into big pieces, lyophilized and ground. The matrix which is regarded as being the most significant consists of the bark of lignified roots of the genus Vitis.

The extraction is obtained with methanol (or alternatively with ethanol) in a volume which is 10 to 20 times the weight of the matrix to be extracted, in an oxygen-free, e.g. saturated with nitrogen, and light-shielded atmosphere at room temperature, with a duration varying according to the matrix. The final extract is concentrated under reduced pressure and at low temperature and taken up with ethyl acetate.

### Example 4

### Purification of viniferine, preparation of the purified extract containing the whole class of these compounds

The concentrated extract obtained is washed with water saturated with an inorganic salt (NaCl), the fraction in ethyl acetate is loaded onto a column prepared with resin of a stirene-divinylbenzene polymer, with grain size between 0.1 and 0.25 mm, then pre-purified through consecutive washings with methanol, methylene chloride, acetone, methanol, water. A volume of water corresponding to about 10 times the volume of the extract to be loaded is left on the head of the column. After the loading the whole is eluted with water, then with pentane-methylene chloride 2:1. Stilbenes are then eluted with ethyl acetate. The product of this selective elution consists of a purified fraction containing the whole class of viniferine, containing both oligostilbenes and stilbenoids. The other polyphenols, strongly adsorbed, are eluted with methanol and/or methanol acidified with strong mineral acid.

In particular, the basic constituents granting a pharmacological interest to this fraction are the oligomers of resveratrol, with particular attention to dimers, trimers and tetramers, both those still containing a stilbene double bond in trans or cis form (oligostilbenes) and those which have lost their stilbene structure during polymerization (stilbenoiids). The same fraction also contains the monomer glucoside trans-resveratrol.

### Example 5

### Purification of resveratrols, preparation of the purified extract, if possible with separation of free forms from glucosidated forms

The raw extract obtained as described above from roots of Polygonum cuspidatum is treated as follows. If only glucosides have to be purified, these can be selectively cold-precipitated in a non polar solvent (hexane would be ideal). Alternatively, the whole fraction can be used in order to recover trans-resveratrol as well. The extract in ethyl acetate, which should be perfectly anhydrous, is reduced to its minimum volume and loaded into the head of a silica preparative column for chromatography (for instance Kieselgel 0.05-0.20 mm), packed in hexane. A suitable volume of chloroform is left above the column. The treatment provides for two subsequent washing and elution sequences with various chloroform-methanol mixtures, I (20:1); II (10:1); III (5:1); IV (2.5:1) respectively. This separation enables an integral recovery of active agents, separated from all major interfering substances, thus obtaining two preparations with very high purity: one contains two different monoglucosides of trans-resveratrol, gathered into the same fraction, and the other one contains free trans-resveratrol. If it is necessary to go as far as the molecular level, purification techniques by preparative chromatography can be applied, in the conditions described in Mattivi et al., *Isolation, characterization and evolution in red wine vinification of resveratrol monomers, J. Agr. Food Chem.*, 1995, 43, 7, 1820-1823.

### Example 6

### Preparation of pure compounds of the family of viniferin

If the starting product consists of the roots of Vitis, the following pure compounds can be obtained by purification: epsilon-viniferine, alfa-viniferine, H-gnetine (which can also be isolated from wood of *Welwitschia mirabilis*), r-viniferine, Ampelopepsin A (which can also be isolated from roots of *Ampelopsis brevipedunculata*) and hopeaphenol.

The final isolation of these pure compounds can be obtained through high-performance liquid chromatography on reverse-phase columns. Phases based on silica functionalized with C18 o C8, or stirene polymers can be used successfully, the latter only with low pressure. A practical example (ideal to separate epsilon-viniferine, H-gnetine and r-viniferine) is the use of a column packed with LiChrospher 100 RP-18, particle diameter 10 microns, eluting with a linear gradient of water and acetonitrile, the latter 30 to 50%.

### Example 7

### Trans-cis isomerization

This reaction can be used both to obtain products which are not commercially available (example: cis-resveratrol) and to shift the balance of mixtures of natural extracts containing both forms, if pharmacologically useful.

Cis isomers can be prepared by means of photoisomerization starting from the correspondent trans isomers. The best conversion yield can be obtained by feeble irradiation in the near spectrum of ultraviolet light or in the spectrum of visible light, which can be carried out in vessels of transparent glass. For instance, the isomerization of a solution containing 0.5 mg/ml of commercial T-Res in ethanol, protected from oxygen by means of degassing in an ultrasound bath, insufflated with nitrogen and then sealed and irradiated at 366 nm, allows to obtain conversion yields around 90% with times of 600 minutes. The conversion can be controlled using the techniques of high pressure liquid chromatography (HPLC) described above, by means of direct injection, stopping the reaction when the desired yield has been reached. Obviously, times and modes have to be adapted according to general practice: stronger irradiations can be used only if faster process controls, known to the person skilled in the art, are applied (direct UV, isocratic HPLC), which is easy anyway in case of single compounds. The alcoholic solution, in our case with ethanol, enables an optimal stability of the preparation, which has to be kept in solution since it has great problems of stability in the solid status.

### Example 8

### Methods of quantitative analysis

As far as the quantification of viniferine, as a mixture from example 4 or 6, is concerned, a volume between 1 and 6 microlitres of extract in ethyl acetate, anhydrified and filtrated at 0.22 micron, is injected on a HPLC system with reverse phase column Hypersil ODS C18, 5 micron, 200x2, 1 mm. The instrumental conditions provide as eluants A=H₃PO₄ 1 mM in water, B=acetonitrile, linear gradient from 100% A, +2% B/min, 0,6 ml/mm flow, 40°C. The detection is obtained with a photodiode detector, detection at 325 nm for oligostilbenes and at 282 nm for stilbenoids. Quantification with external standard method with respect to calibration straight lines with pure reference compounds.

As far as resveratrols are concerned, they can be obtained with two alternative techniques, HPLC or GC. For both techniques quantification is carried out with the internal standard method. The compound we have found to be optimal is trans-4-hydroxy-stilbene, commercially available.

For GC analyses the techniques described in Mattivi et al. *Isolation,* *characterization, and evolution in red wine vinification of resveratrol monomers, J. Agric. Food Chem*., 1995, 43, 7, 1820-1823, are used. A derivatizing agent other than those indicated in the related literature has to be used, consisting of trimethylchlorosilane and hexamethyldisilazane in anhydrous pyridine. These specific conditions are determining to overcome the problems of silanization with bis(trimethylsilil)trifluoroacetamide (BSTFA).

For HPLC the reference method with reverse phase column can be applied, with detection at 310 nm for trans isomers and at 282 for cis isomer (Mattivi F. Solid phase *extraction of trans-resveratrol from wines for HPLC analysis. Zeitschrift für Lebensmittel-Untersuchung und Forschung*, 1993, 196, 522-525). The instrumental conditions are the same as those described above for viniferine. The coefficient of molar extinction of both glucosides is almost the same as the one of the corresponding agfycones, so that they can be added as such after carrying out a correction for molecular weights (Mattivi et al. *Isolation, characterization, and evolution in red wine vinification of resveratrol monomers, J. Agric. Food Chem*., 1995, 43, 7, 1820-1823).

If a preparation for aqueous or hydroalcoholic matrixes is necessary, the injection should be preceded by a pre-purification step on reverse phase cartridges according to Mattivi F. *Solid phase extraction* of *trans-resveratrol from wines for HPLC analysis. Zeitschrift für Lebensmittel-Untersuchung und Forschung*, 1993, 196, 522-525. In addition to what is contained in the aforesaid document, it has been verified that the method is suitable for all monomer stilbenes, both free and glucosides, and that the eluted fraction of ethyl acetate can be dried under a light flow of nitrogen, in case it is necessary to carry out the exchange in another solvent and/or to further reduce the limits of quantification.

Said techniques can be used for instance to control the stability of the active agent in culture mediums, and therefore to support in vitro experimental models which are going to be described in the following examples. For instance, the culture for means described in ex. 10 it is possible to find halving times of 38 h for cis-resveratrol, which is still present in an amount of about 25% at the end of the test (72 h), whereas the halving time of 28 h is still high for trans-resveratrol, which is not present as such after 72 h.

### Example 9

### Methods of qualitative-analysis

The main experiments which are necessary for univocal characterization at molecular level are here recalled according to the technique they use:
Ultraviolet spectroscopy: in a UV spectrophotometer with static cell the double measurement is carried out in absolute ethanol and with the addition of sodium ethylate (Hillis W.E., Ishikura N. 1968 *J. Chromatogr.,* 32, 323). In a photodiodes detector coupled with HPLC, according to Mattivi F., Raniero F. *Relationship between UV spectra and molecular structure of resveratrol oligomers. Polyphenols Communications* 96, 125-126. Given y=A230/A236, the number of stilbene units in the oligomers, i.e. x, is obtained with the equation y=0.657x - 0.065.
Nuclear magnetic resonance: 1 H in NMR spectrometer at 400 MHz or higher, 13C at 100 MHz or higher, in acetone hexadeuterated with TMS as reference. As an alternative, resonances of the methyl group of hexadeuterated acetone can be used as reference (deltaH=2.04 deltaC=29.8). For the specific compounds, according to the requirements, various measuring experiments can be applied, e.g. homonuclear DEPT, 1 H COSY, 1 H COSY long range, double quantum filtered COSY, heteronuclear HECTOR and heteronuclear long range 1H-13C(13C)-GARP decoupling experiments, HMQC, HMBC, NOE difference spectra. Optimal concentrations for measurements in 5 mm probes are 15 mg in 0.5 ml for proton experiments, and 80 mg in 0.5 ml for carbon experiments.
Mass spectrometry, in particular FAB-MS registered in negative modality, in glycerol matrix.
Infrared spectrometry: registered in KBr tablets.
Acetylation of free hydroxyls, in the conditions indicated by Koenig et al. (1987, *Phytochemistry*, 26, 2, 423-427).

### Example 10

Experimental models for the study of functional activities of lymphocytes deriving from human peripheral blood

### Cell separation

Mononucleated cells of human peripheral blood (PBMC) deriving from healthy subjects have been obtained by separation on gradient (Ficoll-Hypaque) of cells deriving from buffy coat.

### Cytofluorometric analysis of intracytoplasmatic cytokine levels

*In vitro* production of cytokines induced by activation with monoclonal anti-CD3 antibody carried out by the various T cell underpopulations in PBMC has been evaluated by means of a simultaneous cytofluorometric determination of the intracytoplasmatic cytokine content and of the expression of surface antigens in every single cell. By using said method it has been possible to evaluate the production of cytokines by single cell under-populations without previously physically separating them, and therefore in presence of the interactions in the production of cytokines which take place in physiological systems. The execution of said method is made possible by the use of saponin as reversible permeabilizing agent of cell membrane. In short, PBMC have been resuspended in a concentration of 1x10⁶/ml, activated by treatment with anti-CD3 (10 ng/ml) and incubated with various doses of resveratrol. After 3 days of culture at 37°C in a CO₂ incubator, the cells have been treated with anti-CD28 (2 ng/ml) and with resveratrol in the same doses previously used. After further 3 days of treatment, the cells have been incubated with a fluorescent monoclonal antibody specific for a surface antigen (CD4 or CD8), resuspended in a medium with the addition of 0.3% saponin, and marked with an optimal dose of a mixture of anti-cytokine monoclonal antibodies conjugated with various fluorochromes. The samples thus treated have then undergone cytofluorometric analysis using a cytofluorometer FACscan (BD).

### Determination of the expression of surface antigens

PBMC have been resuspended in a concentration of 1x10⁶/ml and incubated for different periods of time with various doses of resveratrol. The cells have then been incubated with one or more fluorescent monoclonal antibodies, each being specific for a surface antigen (CD4, CD8, CD16 and CD95), and they have undergone a cytofluorometric analysis using a cytofluorometer FACscan (BD).

### Determination of apoptosis by flow cytofluorometry

PBMC resuspended in a concentration of 1x10⁶/ml have been fixed in acetone/methanol 1:4, treated with 100 KU/ml of RNase and marked with propydium iodide (50 mcg/ml). The cells have then been analyzed by means of flow cytofluorometry using a cytometer FACscan.

### Determination of apoptosis by confocal microscopy

PBMC resuspended in a concentration of 1x10⁶/ml have been fixed in 4% paraformaldehyde, treated with 100 KU/ml of RNase and marked with propydium iodide. The cells have then been analyzed by means of observation with a confocal microscope (LEIKA TCS 4D).

### Determination of cell proliferation and DNA synthesis

Cell replication has been evaluated by a cellular count carried out using a Coulter Counter. DNA synthesis has been measured by the test of incorporation of bromodeoxyuridine (BrdU) evaluated by flow cytometry using a cytometer FACscan (Becton-Dickinson).

### Determination of cell mortality

Cell mortality has been evaluated by a cellular count in trypan blue and/or by flow cytometry using a cytometer FACscan (Becton-Dickinson), determining the vitality of unfixed cells which have been marked with propydium iodide (PI).

### Determination of Natural Killer activity (NK)

PBMC in a concentration of 2x10⁶ cells/ml have been treated in vitro with different doses of resveratrol for 18 h. At the end of the incubation the cells have been tested for their natural cytotoxic activity (NK) against the human erythroleukemic line K 562. The cytotoxic activity of the effect-producing cells incubated for 4 hours at 37°C with K562 has been determined using a cytotoxicity standard test based on the release of ⁵¹Cr by target cells.

### Determination of the cytotoxic activity of T lymphocytes (CTL)

PBMC in a concentration of 2x10⁶ cells/ml have been stimulated in vitro for 5 days at 37°C with irradiated MT-2 cells (a line of human T cells deriving from blood of umbilical cord infected with HTVL-1), and simultaneously treated with different doses of resveratrol. At the end of the incubation the cells have been tested for their cytotoxic activity against MT-2 cells using a cytotoxicity standard test based on the release of ⁵¹Cr by target cells.

### Example 11

### Effects of resveratrol on the production of cytokines in activated human lymphocytes

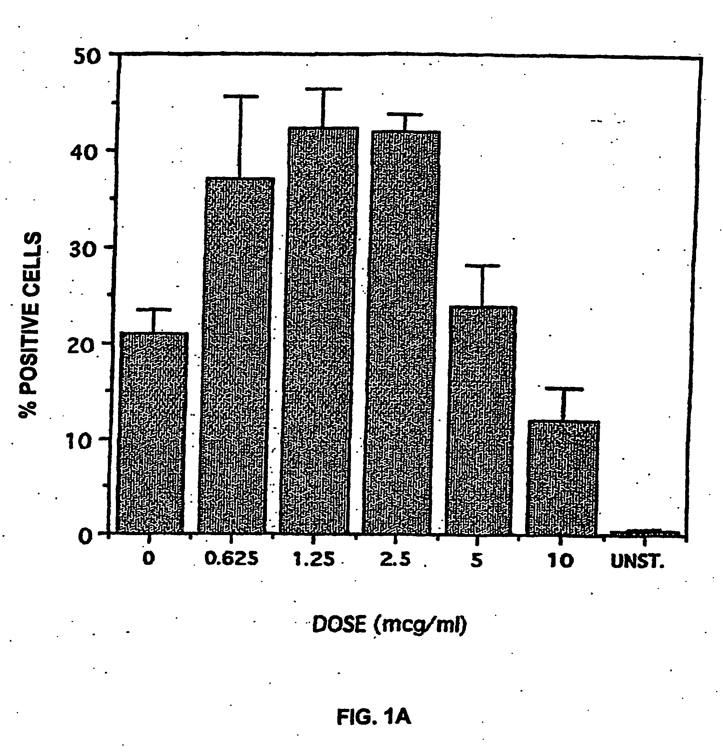

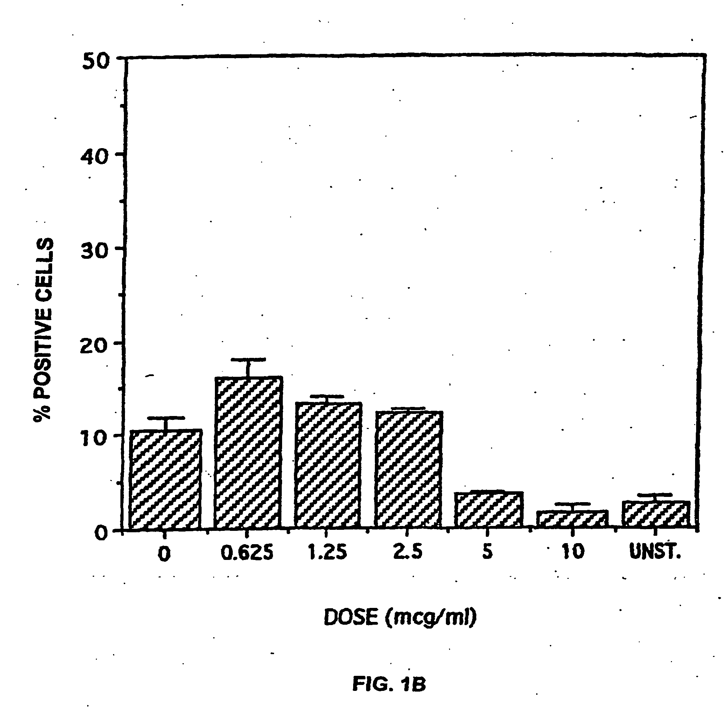

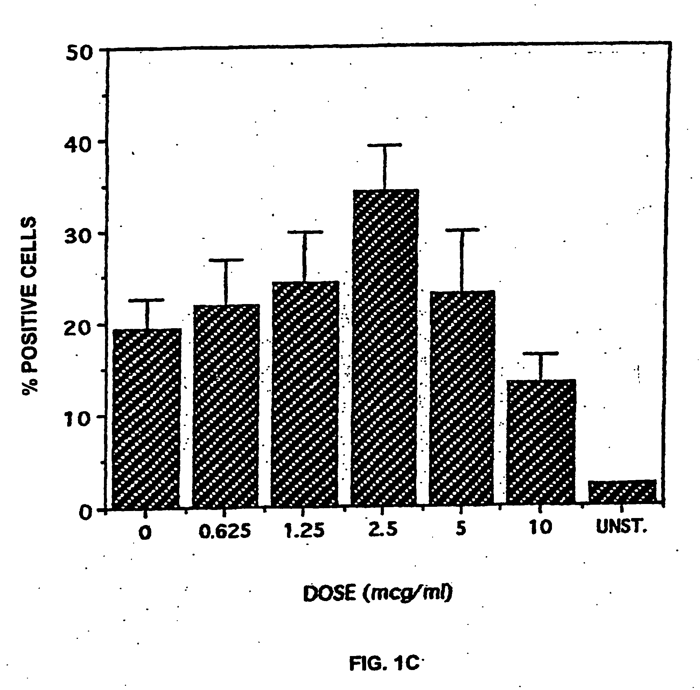

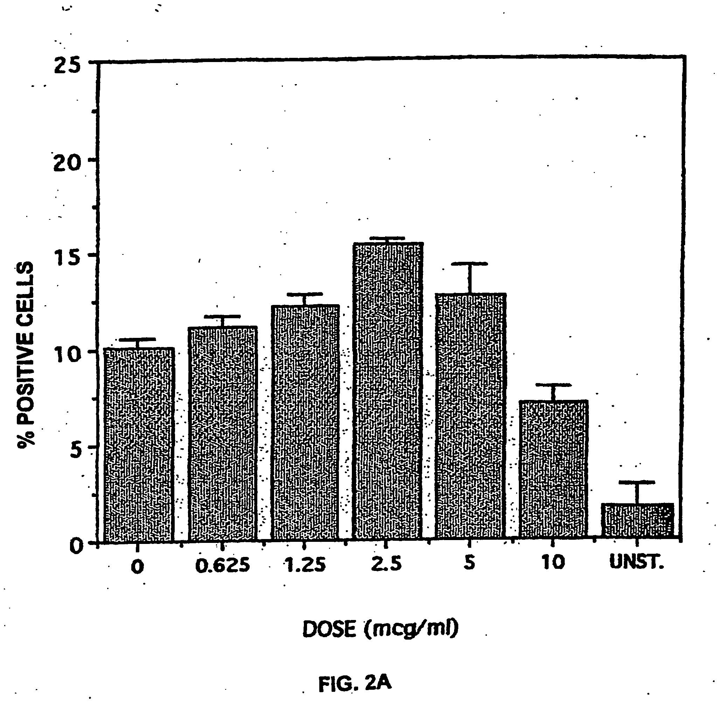

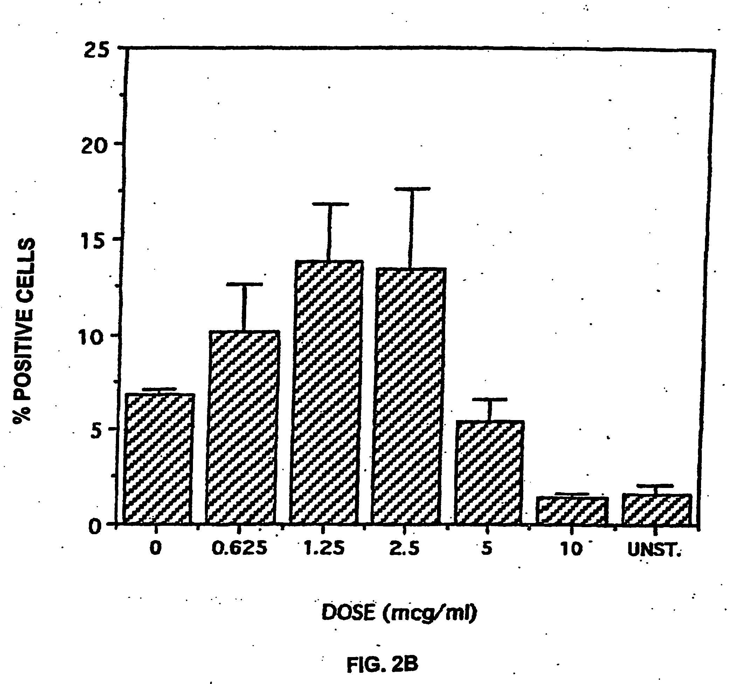

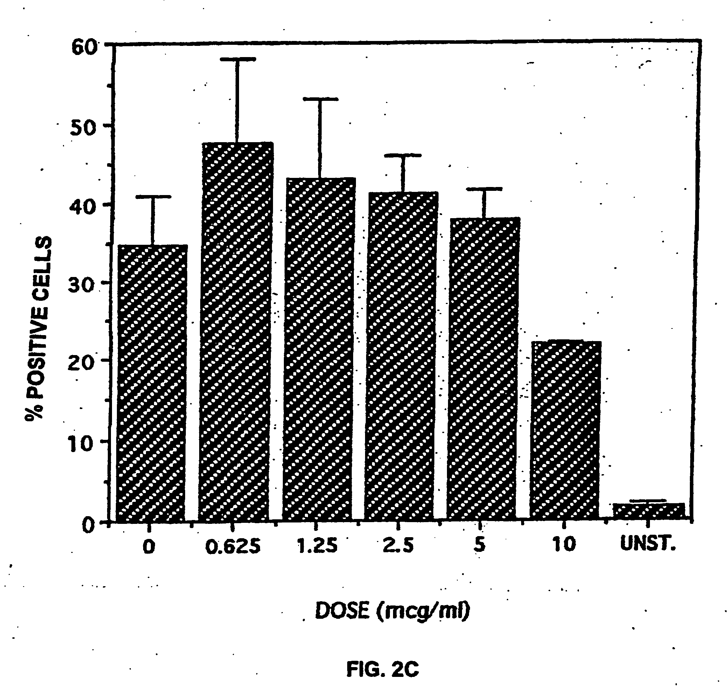

Mononucleated cells of human peripheral blood (PBMC) have been activated by incubation with anti-CD3 + anti-CD28 and then incubated with different doses of resveratrol (or DMSO as control) as described above. Non-activated cells deriving from the same buffy coat have been used as control. The percentage of T lymphocytes CD4+ and CD8+ positive for IL2, IL4 and IFN-g has then been analyzed by means of flow cytofluorometry. The results of the experiments have shown that in a dose range 0.625-2.5 mcg/ml resveratrol induces in the cells activated with anti-CD3 + anti-CD28 a significant increase, with respect to controls, of the percentage of T cells CD8+ positive for intracellular IFN-g, IL2 and IL4 (and therefore activated to synthesize the aforesaid cytokines) (Fig. 1). The activity peak varies according to the examined cytokine, and precisely: 1.25-2.5 for IFN-g (Fig. 1A), 0.625 for IL2 (Fig-. 1B) and 2.5 for IL4 (Fig. 1C). At a dose of 5 and more sigrtificantly at a dose of 10 mcg/ml the effect of resveratrol resulted in an inhibition of the percentage of cells which can synthesize all tested cytokines (Fig. 1A, 1 B, 1 C). Similar results have been obtained for T cells CD4+ (Fig. 2), though with differences as far as the peak of stimulating activity is concerned, which was 2.5, 1.25-2.5 and 0.625 for IFN-g (Fig. 2A), IL2 (Fig. 2B) and IL4 (Fig. 2C) respectively. These results clearly show that resveratrol can modulate (both in the sense of a stimulation at lower doses and of an inhibition at higher doses) the production of cytokines in activated T cells CD8+ and CD4+, and therefore thanks to said effect it can be used as drug with immunomodulating activity.

### Example 12

### Effects of resveratrol on DNA replication and synthesis of activated human lymphocytes

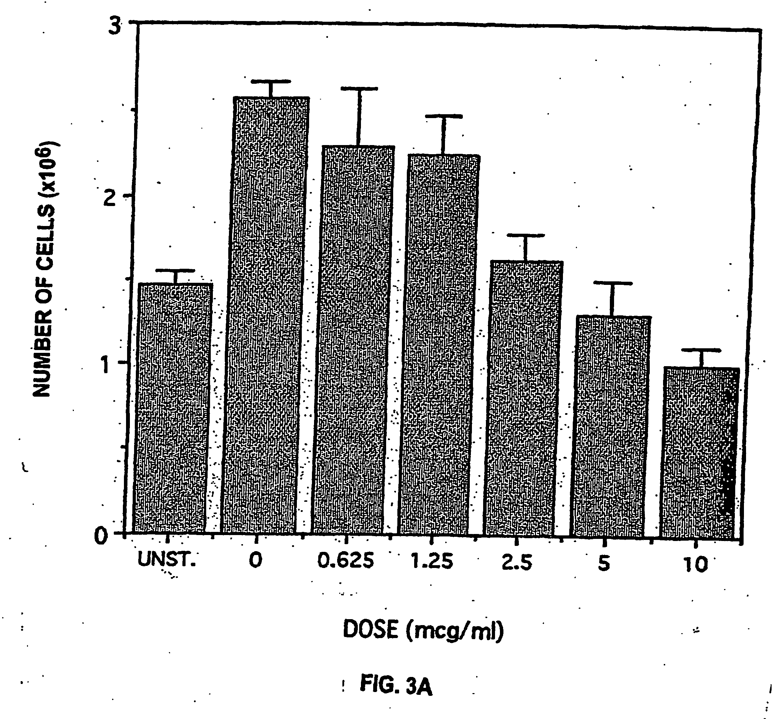

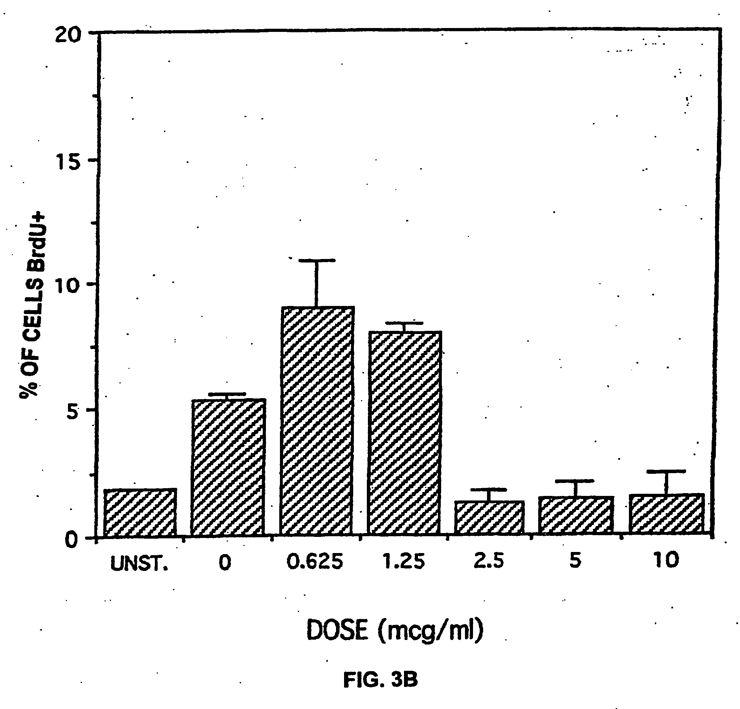

Mononucleated cells of human peripheral blood (PBMC) have been activated by incubation with anti-CD3 + anti-CD28 and then incubated with different doses of resveratrol (or DMSO as control) as described above. Non-activated cells deriving from the same buffy coat have been used as control. The results of the experiments have shown that at doses of 0.625 and 1.25 mcg/ml resveratrol induces a statistically significant increase both in the number (Fig. 3A) and in the ability to synthesize DNA (Fig. 3B) of treated cells, and therefore an increase in their proliferative ability. On the contrary, in a dose range of 2.5-10 mcg/ml the treatment with resveratrol induces a dose-dependent inhibition of cell proliferation (Figs. 3A and 3B).

### Example 13

### Effects of resveratrol on apoptosis and mortality of activated human lymphocytes

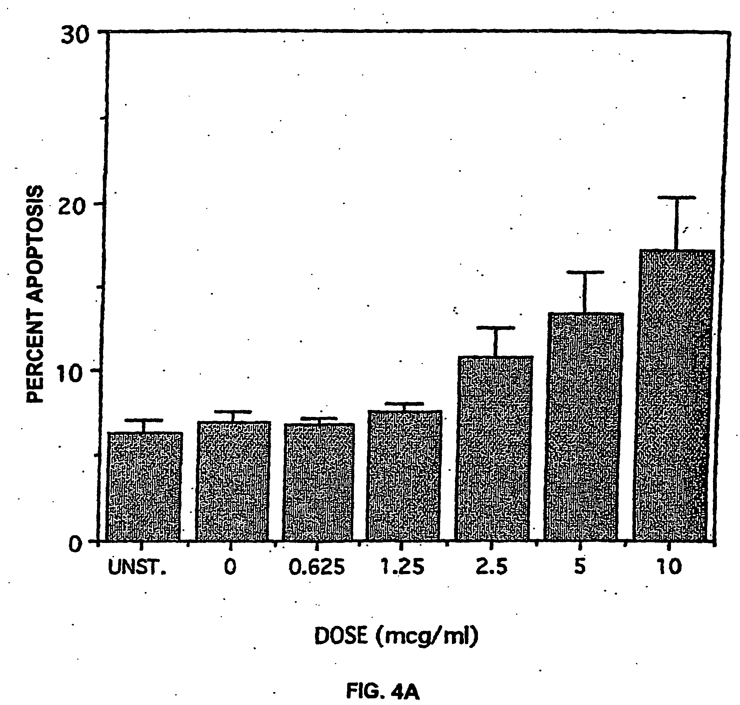

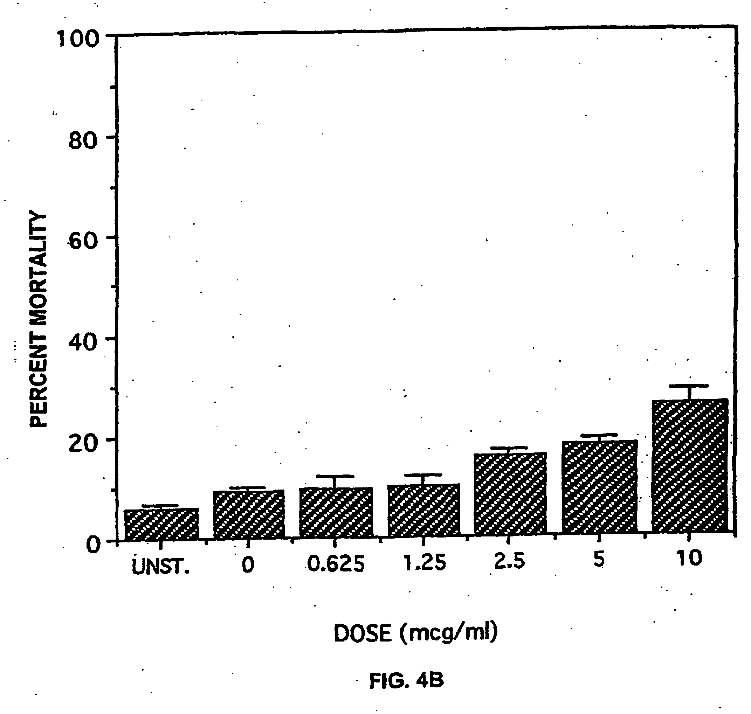

Mononucleated cells of human peripheral blood (PBMC) have been activated by incubation with anti-CD3 + anti-CD28 and then incubated with different doses of resveratrol (or DMSO as control) as described above. Non-activated cells deriving from the same buffy coat have been used as control. The results of the experiments have shown that at doses of 0.625 and 1.25 mcg/ml resveratrol does not have any effect on the apoptosis of activated cells (Fig. 4A). On the contrary, in a dose range of 2.5-10 mcg/ml the treatment with resveratrol induces a dose-dependent increase of the percentage of apoptotic cells (Fig. 4A). Similarly, resveratrol in doses of 0.625 and 1.25 mcg/ml does not have any effect on the mortality of activated cells (Fig. 4B). On the contrary, in a dose range of 2.5-10 mcg/ml the treatment with resveratrol induces a dose-dependent increase of mortality (Fig. 4B).

### Example 14

### Effects of resveratrol on Natural Killer activity (NK) of lymphocytes deriving from human blood

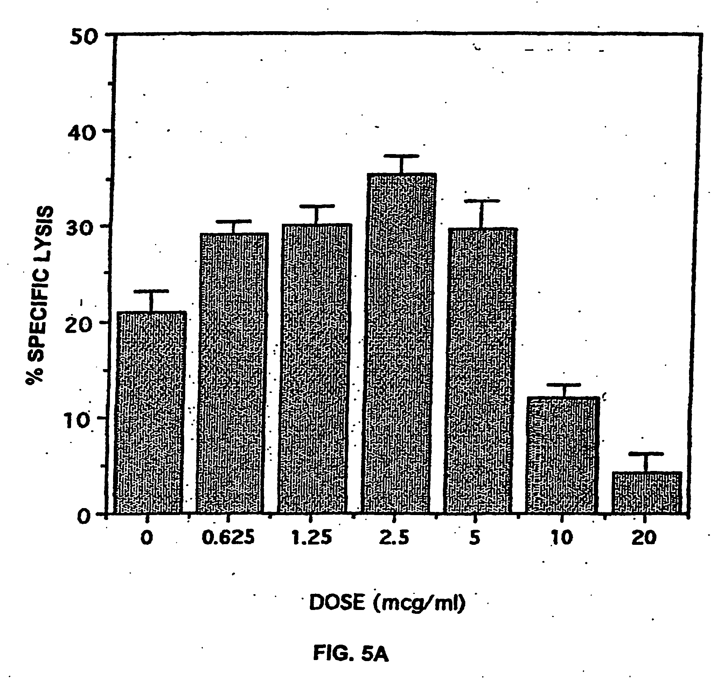

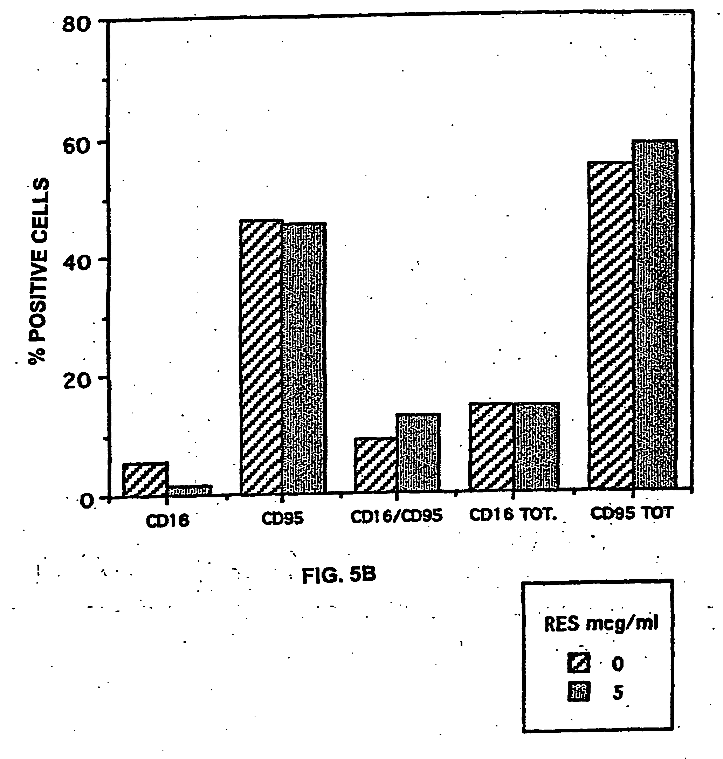

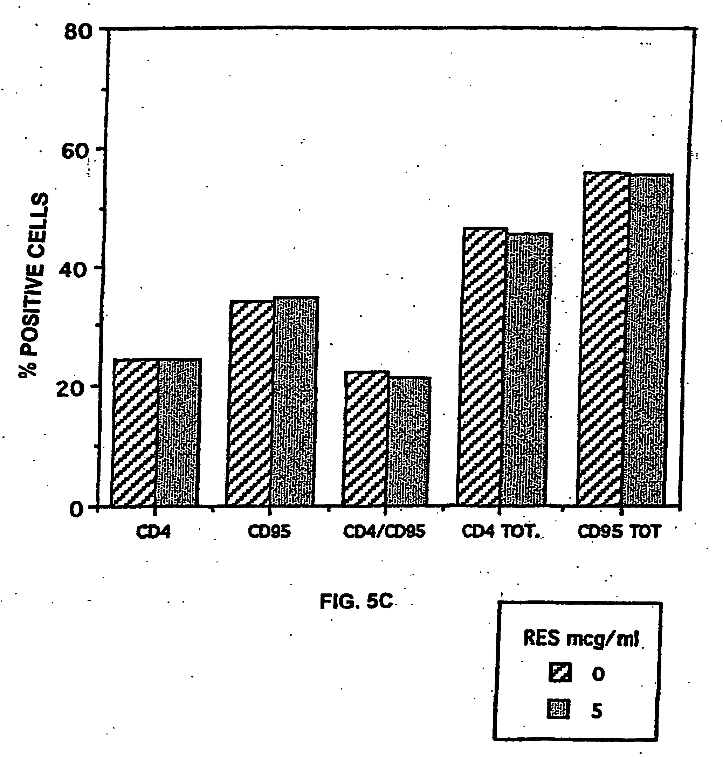

Mononucleated cells of human peripheral blood (PBMC) have been incubated for 18 h with resveratrol in a concentration of 0.625, 1.25, 2.5, 5, 10 and 20 mcg/ml. At the end of the incubation the cells have been tested for their natural cytotoxic activity (NK) against the human erythroleukemic line K 562. The results have shown (Fig. 5A) that in a dose range of 0.625-5 mcg/ml resveratrol induces a statistically significant increase of NK activity. Said significant increase (Fig. 5B), after the treatment with resveratrol in a dose of 5 mcg/ml, of cells which can co-express CD16 and CD95 (CD16+/CD95+ cells) strongly hints at the specific stimulating activity on the parameter taken into consideration of the examined substance, presumably through the stimulation of said cells. Said specificity is also shown in that the treatment of lymphocytes with resveratrol at the same dose does not induce any variation in the percentage of CD4+/CD95+ cells (Fig. 5C). These results clearly show that resveratrol, possibly through the activation of CD16+/CD95+ cells, can perform an immunostimulating activity towards NK response and can therefore strengthen the natural cytotoxic response.

On the contrary, at doses of 10 and 20 mcg/ml resveratrol induces an inhibition of NK activity.

### Example 15

### Effects of resveratrol on cytotoxic activity of T lymphocytes (CTL) deriving from human blood

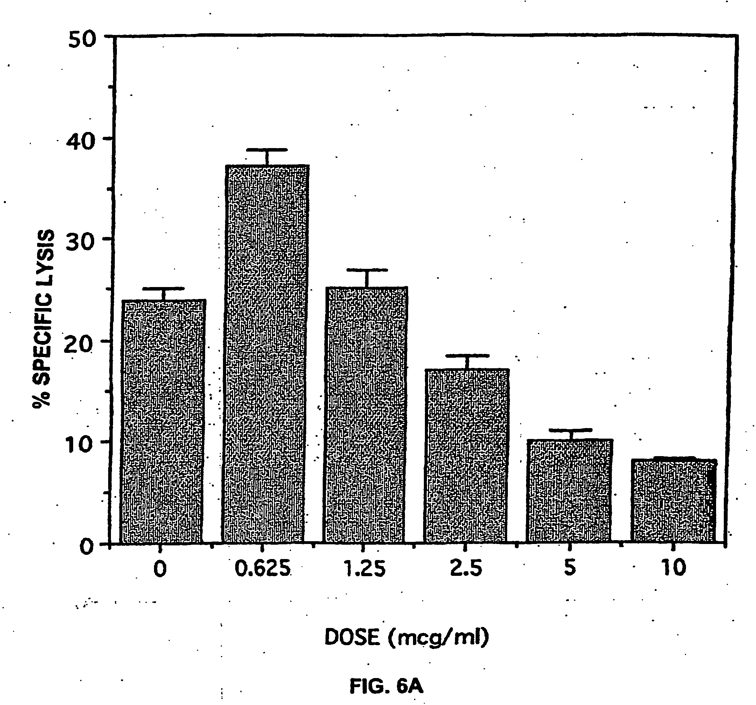

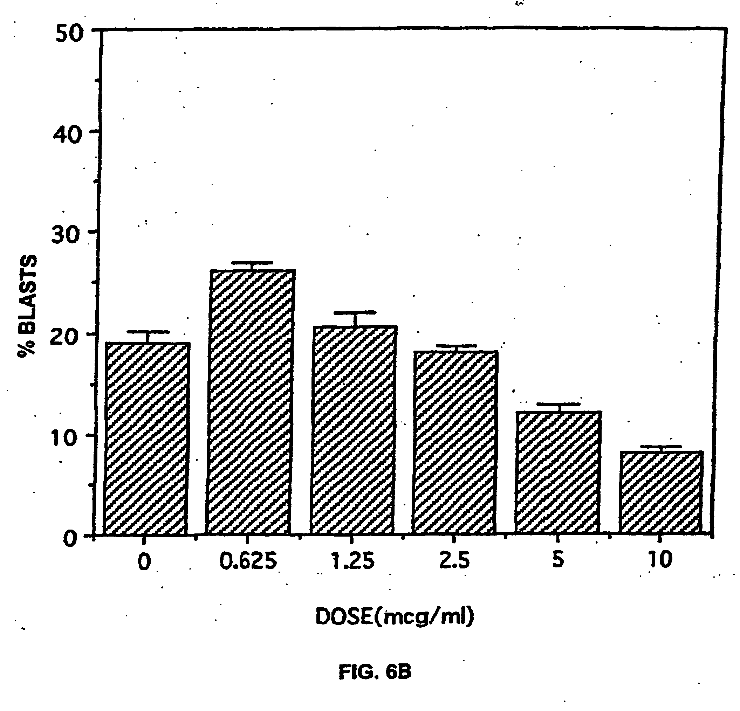

Mononucleated cells of human peripheral blood (PBMC) have been incubated with resveratrol in a concentration of 0.625, 1.25, 2.5, 5 and 10 mcg/ml in presence of MT-2 cells (line of human T cells deriving from blood of umbilical cord infected with HTLV1) for 5 days at 37°C. At the end of the incubation the cells have been tested for their cytotoxic activity against said MT-2 cells. The results of the experiments have shown that at a dose of 0.625 mcg/ml resveratrol induces an increase of cytotoxic activity of T lymphocytes (Fig. 6A) and an increase in the number of blast cells (Fig. 6B). On the contrary, in a dose range of 2.5-10 mcg/ml resveratrol induces an inhibition both of cytotoxic activity (Fig. 5A) and of the number of blast cells (Fig. 5B).

### Example 16

### Effects of resveratrol on the percentage of T lymphocytes CD4+ and CD8+ in activated human PBMC

Mononucleated cells of human peripheral blood (PBMC) have been activated by incubation with anti-CD3 + anti-CD28 and then incubated with different doses of resveratrol (or DMSO as control) as described above. The results of the experiments have shown that resveratrol induces a dose-dependent decrease of the percentage of activated CD8+ cells in a dose range of 2.5-10 mcg/ml. On the contrary, it does not induce any variation at lower doses or on the percentage of CD4+ cells.

## Claims

1. Use of at least one compound selected in the group of: Trans-Resveratrol, Cis-Resveratrol, glucosidated Cis-Resveratrol, ε-viniferine, H-gnetine, r-2-viniferine, r-viniferine, hopeaphenol, Ampelopepsin A and glucosidated Trans-Resveratrol and corresponding mixtures as active agent for the manufacture of a medicament for treating at least one of the following affections: primary immunodeficiency in children and adults; secondary immunodeficiencies deriving for instance from undernourishment, lymphoproliferative illnesses, infections induced by virus, bacteria, fungi and animal parasites, surgery interventions, radiotherapy and/or chemotherapy treatments, drug administration, burns and nephrotic syndrome.

2. Use of at least one compound selected in the group of: Trans-Resveratrol, Cis-Resveratrol, glucosidated- Cis-Resveratrol, ε-viniferine, H-gnetine, r-2-viniferine, r-viniferine, hopeaphenol, Ampelopepsin A and glucosidated Trans-Resveratrol and corresponding mixtures as active agent for the manufacture of a medicament for treating at least one affection selected among autoimmune illnesses such as rheumatoid arthritis, uveitis, psoriasis; transplant reject.

3. Use according to claims 1 or 2 in combination with a pharmaceutically active therapeutic agent selected in the group of anti-tumor compounds such as antimetabolites, intercalating agents, mitosis inhibitors or other cytotoxic inhibitors such as platinum coordination complexes, vinca alkaloids, urea substitutes, L-asparaginase, corticoadrenal inhibitors.

4. Use according to claims 1-3 wherein the medicament is administered in form of pharmaceutical acceptable salt, ester, amide, prodrug or similar, and corresponding combinations.

5. Use according to claims 1-3 wherein the medicament is in form of tablets, suppositories, pills, capsules, powders, liquids, suspensions, creams, ointments or lotions.

6. Use according to claims 1-3 wherein the medicament is to be administered by oral, parenteral (subcutaneous, intravenous, intramuscular injection), transdermic, rectal, nasal, buccal, topical way or by a controlled-release implant.

7. Use according to claims 1-3 wherein the medicament is to be administered at doses between 0.0001 and 20 mg/kg/die of the active agent.

## Patentansprüche

1. Verwendung von mindestes einer Verbindung ausgewählt aus der Gruppe enthaltend: Trans-Resveratrol, Cis-Resveratrol, glukosidiertes Cis-Resveratrol, ε-Vniferin, H-Gnetin, r-2-Viniferin, r-Viniferin, Hopeaphenol, Ampelopepsin A und glukosidiertes Trans-Resveratrol und entsprechende Mischungen davon als aktives Agens für die Herstellung eines Medikamentes zur Behandlung von mindestens einem der folgenden Krankheitszustände: primäre Imrnunodefizienz bei Kindern und Erwachsenen, sekundäre Immunodefizienzen verursacht beispielsweise durch Unterernährung, lymphoproliferative Erkrankungen, Infektionen hervorgerufen durch Vren, Bakterien, Pilze und Tierparasiten, chirurgische Eingriffe, Strahlen- und/oder Chemotherapien, Medikamentenverabreichung, Verbrennungen und nephrotisches Syndrom.

2. Verwendung von mindestes einer Verbindung ausgewählt aus der Gruppe enthaltend: Trans-Resveratrol, Cis-Resveratrol, glukosidiertes Cis-Resveratrol, E-Viniferin, H-Gnetin, r-2-Viniferin, r-Viniferin, Hopeaphenol, Ampelopepsin A und glukosidiertes Trans-Resveratrol und entsprechende Mischungen davon als aktives Agens für die Herstellung eines Medikamentes zur Behandlung von mindestens einem der folgenden Leiden aus der Gruppe der Autoimmunerkrankungen, wie beispielsweise rheumatoide Arthritis, Uveitis, Psoriasis, Abstoßung von Transplantaten.

3. Verwendung gemäß einem der Ansprüche 1 oder 2 in Kombination mit einem pharmazeutisch aktiven therapeutischen Agens ausgewählt aus der Gruppe der Antitumor-Verbindungen, wie beispielsweise Antimetaboliten, interkalierende Agenzien, Mitoseinhibitoren oder andere zytotoxische Inhibitoren wie Platinkomplexe, Vinka-Alkaloide, Surrogate für Harnstoff, L-Asparaginase, kortikoadrenale inhibitoren.

4. Verwendung gemäß den Ansprüchen 1-3, in welchen das Medikament in Form von pharmazeutisch akzeptablem Salz, Ester, Amid, Prodrug oder ähnlichem verabreicht wird und in entsprechenden Kombinationen davon.

5. Verwendung gemäß den Ansprüchen 1-3, in welchen das Medikament in Form von Tabletten, Suppositorien, Pillen, Kapseln, Pulvem, Flüssigkeiten, Suspensionen, Cremes, Salben oder Lotionen vorliegt.

6. Verwendung gemäß den Ansprüchen 1-3, in welchen das Medikament über die orale, parenterale (subkutane, intravenöse, intramuskuläre Injektion), transdermale, rektale, nasale bukkale oder topische Route zu verabreichen ist oder über ein Implantat zur kontrollierten Freisetzung.

7. Verwendung gemäß den Ansprüchen 1-3, in welchen das Medikament in aktiven Wirkstoffdosierungen von 0,0001 bis 20 mg/kg/die verabreicht wird..

## Revendications

1. Utilisation d'au moins un composé choisi dans le groupe consistant en : le trans-resvératrol, le cis-resvératrol, le cis-resvëzatral glucosidé, la ε-viniférine, la H-gnétine, la r-2-viniférine, la r-viniférine, l'hopéaphénol, l'ampélopepsine A et le trans-resvëratrol glucosidé et des mélanges correspondants en tant qu'agent actif pour la fabrication d'un médicament pour traiter au moins l'une des affections suivantes : l'immunodëficience primaire chez l'enfant et l'adulte ; les immunadéficiences secondaires dues, par exemple, à la malnutrition, des maladies lymphoprolifératives, des infections induites par des virus, des bactéries, des champignons et des parasites animaux, des interventions chirurgicales, des traitements de radiothérapie et/ou de chimiothérapie, l'administration de médicament, les brûlures et le syndrome néphrétique.

2. Utilisation d'au moins un composé choisi dans le groupe consistant en : le trans-resvératrol, le cis-resvératrol, le cis-resvératrol glucosidé, la ε-viniférine, la H-gnétine, la r-2-viniférine, la r-viniférine, l'hopéaphénol, l'ampélvpepsine A et le trans-resvératrol glucosidé et des mélanges correspondants en tant qu'agent actif pour la fabrication d'un médicament pour traiter au moins l'une des affections choisies parmi des maladies auto-immunes telles que la polyarthrite rhumatoïde, l'uvéite, le psoriasis ; le rejet de greffe.

3. Utilisation selon les revendications 1 ou 2 en combinaison avec un agent thérapeutique pharmaceutiquement actif choisi dans le groupe consistant en des composés antitumeurs tels que des antimétabolites, des agents intercalants, des inhibiteurs de mitose ou d'autres inhibiteurs cytotoxiques tels que des complexes de coordination de platine, des alcaloïdes de pervenche, des substituts de l'urée, la L-asparaginase, des inhibiteurs corticosurrénaux.

4. Utilisation selon les revendications 1 à 3 dans laquelle le médicament est administré sous la forme de sel, d'ester, d'amide, de promédicament ou similaire pharmaceutiquement acceptable, et de combinaisons correspondantes.

5. Utilisation selon les revendications 1 à 3 dans laquelle le médicament est sous la forme de comprimés, de suppositoires, de pilules, de gélules, de poudres, de liquides, de suspensions, de crèmes, de pommades ou de lotions.

6. Utilisation selon les revendications 1 à 3 dans laquelle le médicament est destiné à être administré par voir orale, parentérale (injection sous-cutanée, intraveineuse, intramusculaire), transdermique, rectale, nasale, buccale, locale ou par un implant à libération contrôlée.

7. Utilisation selon les revendications 1 à 3 dans laquelle le médicament est destiné à être administré à des doses comprises entre 0,0001 et 20 mg/kg/die de l'agent actif.
